# EUROPEAN PATENT SPECIFICATION

(11) **EP 0 830 600 B1**
(45) Date of publication and mention of the grant of the patent: **27.08.2003**
(21) Application number: 96918089.2
(22) Date of filing: 05.06.1996
(51) Int. Cl.: G01N 33/53, C12P 21/06, C12N 5/00, C12N 1/20, C12N 15/00, C07K 1/00, A61K 38/00, G01N 33/50, C07K 14/72

(54) **FUNCTIONAL BIOASSAY FOR G-PROTEIN COUPLED RECEPTOR AGONISTS AND ANTAGONISTS**
FUNKTIONELLER BIOASSAY FÜR AN G-PROTEIN GEKOPPELTE REZEPTORAGONISTEN UND ANTAGONISTEN
DOSAGE BIOLOGIQUE FONCTIONNEL POUR LES AGONISTES ET ANTAGONISTES DES RECEPTEURS COUPLES DE PROTEINE G

(30) Priority: 07.06.1995 US 479803
(43) Date of publication of application: 25.03.1998
(73) Proprietor: Praecis Pharmaceuticals Incorporated, Cambridge, MA 02139-1572 (US)
(72) Inventor: ISRAEL, David, I., Concord, MA 01742 (US); MOLINEAUX, Christopher, J., Brookline, MA 02146 (US)
(74) Representative: Kirkham, Nicholas Andrew
(86) International application number: US9608895
(87) International publication number: WO96041169

(56) References cited:
- EP-A- 0 678 577
- WO-A-93/03137
- WO-A-94/00590
- WO-A-94/09116
- GB-A- 2 283 239
- SCIENCE, 29 July 1988, Vol. 241, JULIUS et al., "Molecular Characterization of a Functional cDNA Encoding the Serotonin 1c Receptor", pages 558-564.
- JOURNAL OF BIOLOGICAL CHEMISTRY, 03 June 1994, Vol. 269, No. 22, HAWES et al., "Inhibition of G Protein-Coupled Receptor Signaling by Expression of Cytoplasmic Domains of the Receptor", pages 15776-15785.
- MOLECULAR PHARMACOLOGY, 1995, Vol. 48, GUAN et al., "Determination of Structural Domains for G Protein Coupling and Ligand Binding in beta3-Adrenergic Receptor", pages 492-498.
- ANALYTICAL BIOCHEMISTRY, 1992, Vol. 206, POTENZA et al., "A Method for Evaluating the Effects of Ligands Upon Gs Protein-Coupled Receptors Using a Recombinant Melanophore-Based Bioassay", pages 315-322.
- JOURNAL OF ANIMAL SCIENCE, July 1994, Vol. 72, No. 7, WEESNER et al., "Rapid Communication: Nucleotide Sequence of Luteinizing Hormone-Releasing Hormone (LHRH) Receptor cDNA in the Pig Pituitary", page 1911.
- PROC. NATL. ACAD. SCI. U.S.A., March 1994, Vol. 91, JAYAWICKREME et al., "Creation and Functional Screening of a Multi-Use Peptide Library", pages 1614-1618.
- PROC. NATL. ACAD. SCI. U.S.A., April 1993, Vol. 90, LIGGETT et al., "Structural Basis for Receptor Subtype-Specific Regulation Revealed by a Chimeric beta3/beta2-Adrenergic Receptor", pages 3665-3669.
- TINS, 1994, Vol. 17, No. 4. LERNER, "Tools for Investigating Functional Interactions Between Ligands and G-Protein-Coupled Receptors", pages 142-146.

## Description

Cellular responses to external stimuli are primarily mediated by receptor based systems. One of the most ubiquitous and sizable transmembrane receptor families, with members observed in organisms from bacteria to yeast to man, is the G-protein coupled receptor (GPCR) family. Receptors of this family associate with a membrane-bound G protein composed of α, β and γ subunits. Following agonist binding to the receptor, a conformational change is transmitted to the G protein, which causes the α-subunit to exchange a bound GDP molecule for a GTP molecule and to dissociate from the βγ-subunits. The GTP-bound form of the α-subunit typically functions as an effector-modulating moiety, leading to the production of second messengers, such as cyclic AMP (e.g., by activation of adenylate cyclase), diacylglycerol or inositol phosphates. Greater than 20 different types of α-subunits are known in man, which associate with a smaller pool of β and γ subunits. Thus, different G-protein coupled receptors may associate with G-proteins having different α, β and/or γ subunits. For reviews on signal transduction by G-protein coupled receptors, see Gilman, A.G. (1987) *Annu. Rev. Biochem*. 56:615-649; Stryer, L. and Bourne, H.R. (1986) *Annu. Rev. Cell. Biol*. 2:391-419; and Bimbaumer, L. (1990) *Annu. Rev. Pharmacol. Toxicol*. 30:675-705.

Most GPCRs are members of the seven transmembrane segment (7 TMS) receptor family. This family is made up of at least several hundred distinct receptors and includes receptors that respond to signals ranging from environmental stimuli, such as photons, odorant molecules and sweet tasting sugars, to molecules involved in intercellular communication, such as biogenic amines, lipids, peptides and glycoproteins, including neurotransmitters, neuromodulators and hormones. Structurally, the receptors of the 7 TMS family are composed of an extracellular amino terminal domain, seven hydrophobic regions that span the cell membrane, six "loop" domains that connect the transmembrane segments (3 intracellular and 3 extracellular) and, for many but not all members, an intracellular carboxy terminal domain. The transmembrane segments of 7 TMS family members exhibit considerable homology, whereas the connecting loops are less conserved, showing high homology only between closely related receptor subtypes. Two of the most extensively studied 7 TMS receptors are rhodopsin, which is expressed in the retinal rod cells and converts light energy into a neurochemical signal (see e.g., Nathans, J. (1987) *Annu. Rev. Neurosci.* 10:163-194) and the β2-adrenergic receptor, which is expressed in most mammalian tissues and activates adenylate cyclase to produce cAMP (see e.g., Dixon, R.A.F. et al. (1986) *Nature* 321:75-79). Additionally, the conserved structure among 7 TMS receptors has allowed for the cloning of many novel genes encoding 7 TMS receptors whose natural ligand and function are yet to be elucidated. These receptors have been referred to as "orphan" receptors (see e.g., Mills, A. and Duggan, M.J. (1993) *Trends in Pharmacological Sciences* 14:394-396). For reviews on 7 TMS receptors, see e.g., Dohlman, H.G. et al. (1991) *Annu. Rev. Biochem.* 60:653-688; and Lefkowitz, R.J. et al. (1993) *Trends in Pharm. Sciences* 14:303-307.

Because of the involvement of G-protein coupled receptors in the regulation of many critically important biological functions, disease conditions may be influenced or determined by the state of activation or blockade of a G-protein coupled receptor. Receptor mutations responsible for human diseases are being elucidated at an increasing rate and may be either loss-of-function mutations or activating mutations. Examples include mutation of the thyrotropin receptor gene, leading to hyperfunctioning thyroid adenomas (see e.g., Parma, J. et al. (1993) *Nature* 365:649-651), mutation of the rhodopsin gene, leading to retinitis pigmentosa (see e.g., Keen, T.J. et al. (1991) *Genomics* 11:199-205; and Robinson, P.R. et al. (1992) *Neuron* 9:719-725), mutation of the luteinizing hormone receptor gene, leading to precocious puberty (see e.g., Shenker, A. et al. (1993) *Nature* 365:652-654) and mutation of the adrenocorticotropin receptor gene, leading to familial glucocorticoid deficiency (see e.g., Clark, A.J.L. et al. (1993) *Lancet* 341:461-462). For a review of the role of GPCRs in disease, see Coughlin, S.R. (1994) *Curr.* Op. *Cell. Biol.* 6:191-197.

Numerous important drugs for the treatment of various disease conditions act by influencing the activity of G-protein coupled receptors. Examples include agonist analogs of gonadotropin-releasing hormone, such as leuprolide, gonadorelin and nafarelin, which have been used to treat prostate and breast carcinomas, uterine leimyomatas, endometriosis, precocious puberty and nontumorous ovarian hyperandrogenic syndrome (see e.g., Pace, J.N. et al. (1992) *Am. Fam. Physician* 44:1777-1782), the cardiac β-adrenergic receptor antagonist propranolol, which has been used to treat hypertension, angina pectoris and psychiatric disorders (see e.g., Nace, G.S. and Wood, A.J. (1987) *Clin. Pharmacokinet.* 13:51-64; Ananth, J. and Lin, K.M. (1986) *Neuropsychobiology* 15:20-27), the pulmonary β2-adrenergic receptor agonist metaproterenol, which has been used as a bronchodilator (see e.g., Hurst, A. (1973) *Ann. Allergy* 31:460-466) and the histamine 2 receptor antagonist cimetidine, which has been used to treat ulcers and idiopathic urticaria (see e.g., Sontag, S. et al. (1984) *N. Engl. J. Med.* 311:689-693; Choy, M. and Middleton, R.K. (1991) *DICP* 25:609-612).

### Summary of the Invention

Given the important role of GPCRs in both normal cellular responses and aberrant disease processes, assays that allow for the identification of agonists or antagonists of GPCRs are highly desirable. This invention provides functional bioassays for identifying agonists or antagonists of a wide variety of GPCRs. The bioassays of the invention are simple, rapid and high-throughput. Moreover, these screening methods enable mammalian GPCRs, and in particular human GPCRs, to be assayed within a mammalian cellular environment Compounds can be tested in these assays singly or, more preferably, in libraries of compounds. Thus, the assays allow for rapid screening of large panels of compounds. Furthermore, the assays of the invention can be applied both to known GPCRs and to "orphan" GPCRs for which a natural ligand is unknown. Thus, the methods of the invention are useful for identifying therapeutically useful agonists or antagonists of known GPCRs as well as for investigating the function of orphan GPCRs.

In the methods of the invention, a population of indicator cells (e.g., mammalian cells) is contacted with a test composition containing one or more test compounds, at least one parameter of cellular metabolism of the indicator cells is measured and the test compound(s) is identified as a receptor agonist or antagonist. The indicator cells express the GPCR of interest, preferably by introduction into the cells of a nucleic acid molecule encoding the GPCR. The test composition can include one or several (e.g., a library) of test compounds. Additionally the test composition may include one or more known receptor agonists or antagonists. For example, a test compound can be identified as a receptor antagonist based upon its ability to alter the effects of a known receptor agonist on the indicator cells when the indicator cells are contacted with both the test compound and the known receptor agonist. Furthermore, the test composition can include one or more agents that alter the metabolism of a second messenger(s) in the indicator cells.

Preferred GPCRs for use in the assays of the invention include luteinizing hormone releasing hormone receptor (LHRH-R; also referred to as gonadotropin releasing hormone receptor (GnRH)), M1 muscarinic receptor and β2-adrenergic receptor (β2-AR). In another embodiment, the GPCR couples to a Gq/11 G-protein (e.g., LHRH-R, acetylcholine, adenosine 1, α-adrenergic, angiotensin, bombesin, bradykinin, C5a, cholycystokinin, endothelin, glutamate, SHT, histamine (H1 subtype), neurotensin, neurokinin, oxytocin, thyrotropin releasing hormone, thyroid stimulating hormone, thromoboxane A2 and vasopressin). In another embodiment, the GPCR couples to a Gs G-protein (e.g., β2-AR, cardiac β-adrenergic, histamine (H2 subtype), thyrotropin, growth hormone releasing factor and adrenocorticotropic hormone). In yet another embodiment, the GPCR is a chimeric receptor composed of at least one ligand binding domain of a first seven transmembrane segment receptor and at least one signal transduction domain of a second seven transmembrane segment receptor such that the chimeric receptor couples to a Gq/11 G-protein in the indicator cells. In still another embodiment, the GPCR is an orphan receptor (i.e., the natural ligand for the G-protein coupled receptor is unknown).

In one embodiment of the screening assay of the invention, the parameter of cellular metabolism that is measured in the indicator cells is viability or proliferation. For example, a test compound can be identified as a receptor agonist based upon its ability to decrease the viability and/or proliferation of the indicator cells when contacted with the indicator cells. Alternatively, a test compound can be identified as a receptor antagonist based upon its ability to increase the viability and/or proliferation of the indicator cells when contacted with the indicator cells and a known receptor agonist. In this embodiment, the GPCR preferably couples to a Gq/11 G-protein (e.g., LHRH-R) or is a chimeric GPCR composed of a signal transduction domain(s) that allows the chimeric GPCR to couple to a Gq/11 G-protein.

In another embodiment of the screening assay of the invention, the parameter of cellular metabolism that is measured in the indicator cells is cellular morphology. For example, a test compound can be identified as a receptor agonist based upon its ability to alter the morphology of the indicator cells when contacted with the indicator cells. Alternatively, a test compound can be identified as a receptor antagonist based upon its ability to restore the indicator cells to their original morphology when contacted with the indicator cells and a known receptor agonist.

The invention further provides methods for preparing a cell that expresses a recombinant G-protein coupled receptor on a membrane of the cell for use as an indicator cell in the screening assays. In this method, a nucleic acid molecule that encodes the receptor is introduced into the cell under culture conditions in which stimulation of the receptor by receptor agonists is inhibited. A stable population of cells expressing a recombinant G-protein coupled receptor in a culture medium under culture conditions in which stimulation of the receptor by receptor agonists is inhibited is also within the scope of the invention.

Nucleic acid molecules encoding chimeric GPCRs and host cells expressing GPCRs are also provided by the invention.

### Brief Description of the Drawings

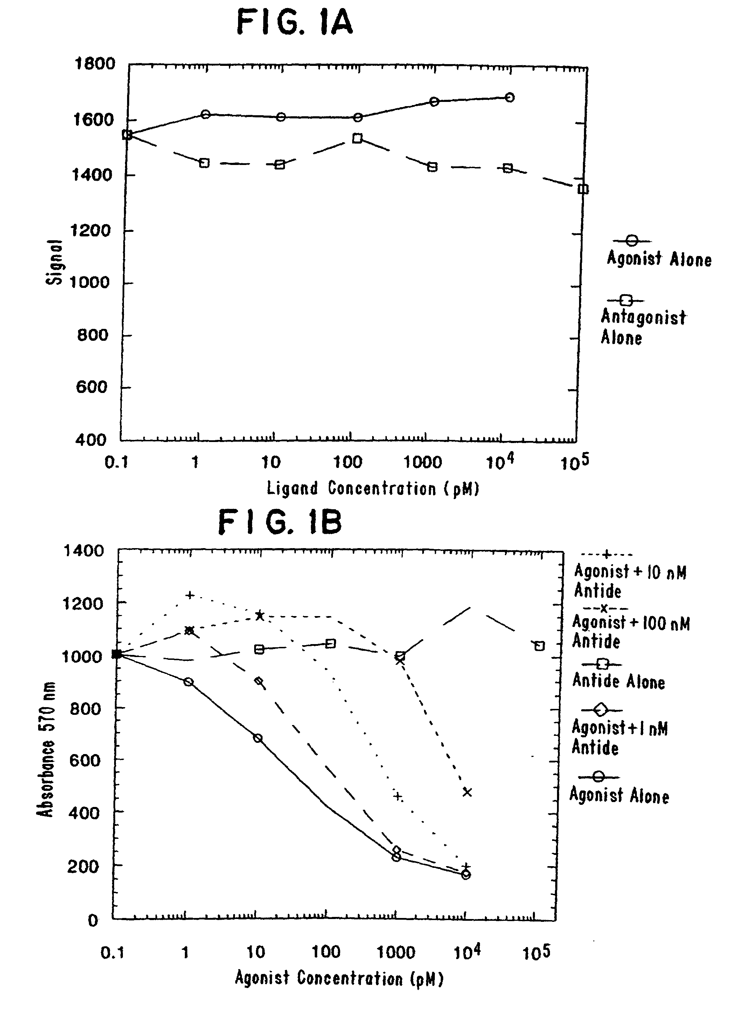

*Figure 1A-B* are graphic representations of the reduction of MTT by CHO cells, either untransfected (panel A) or transfected to express luteinizing hormone releasing hormone receptor (LHRH-R) (panel B), in the presence of increasing concentrations of an LHRH-R agonist alone, increasing concentrations of an LHRH-R antagonist (antide) alone, or for the transfected cells (panel B), increasing concentrations of an LHRH-RR agonist together with 1 nM, 10 nM or 100 nM antagonist.

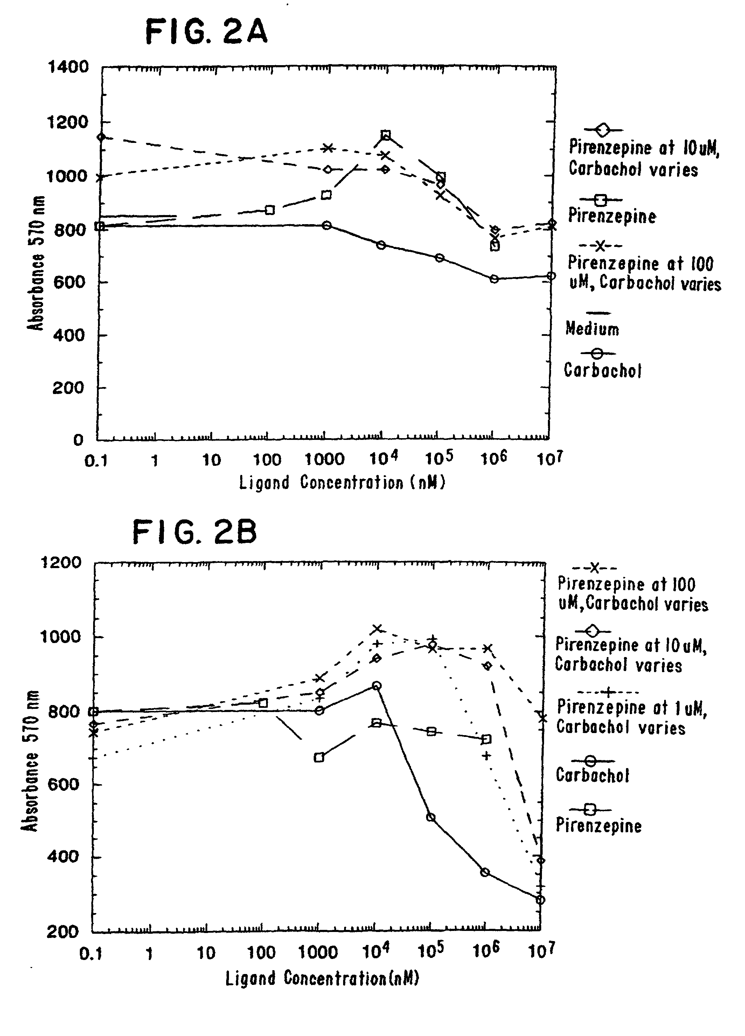

*Figure 2A-B* are graphic representations of the reduction of MTT by CHO cells, either untransfected (panel A) or transfected to express M1 muscarinic receptor (panel B), in the presence of increasing concentrations of an M1 muscarinic receptor agonist (carbachol) alone, increasing concentrations of an M1 muscarinic receptor antagonist (pirenzepine) alone, or increasing concentrations of agonist together with 1 µM (panel B only), 10 µM or 100 µM antagonist.

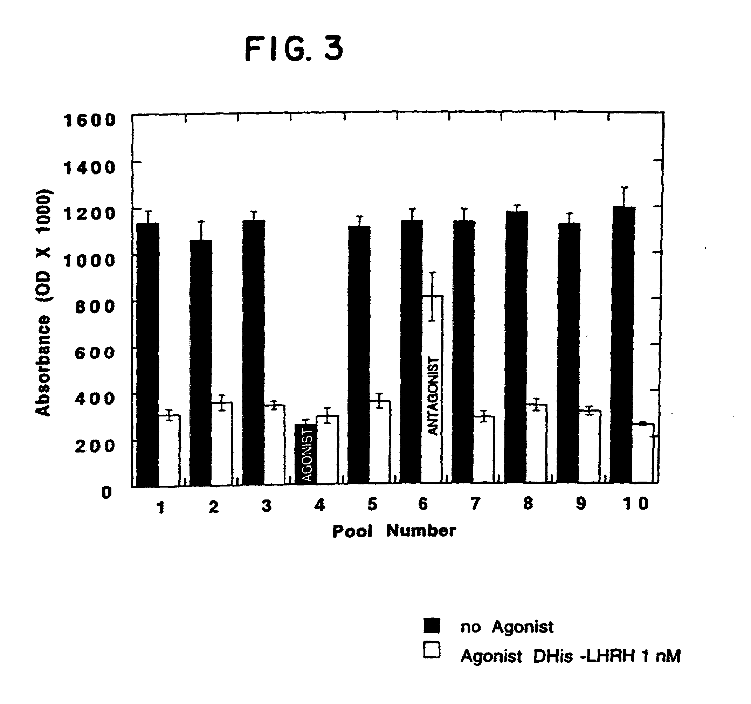

*Figure* 3 is a graphic representations of the reduction of MTT by CHO cells transfected with the LHRH receptor in as assay in which a combinatorial peptide library was incubated with the CHO cells in either the presence (filled bars) or absence (open bars) of the LHRH agonist D-His⁶-LHRH (at 1 nM). In two samples, a known LHRH agonist or antagonist was "spiked" into the sample to demonstrate that both agonists and antagonists can be identified using this assay.

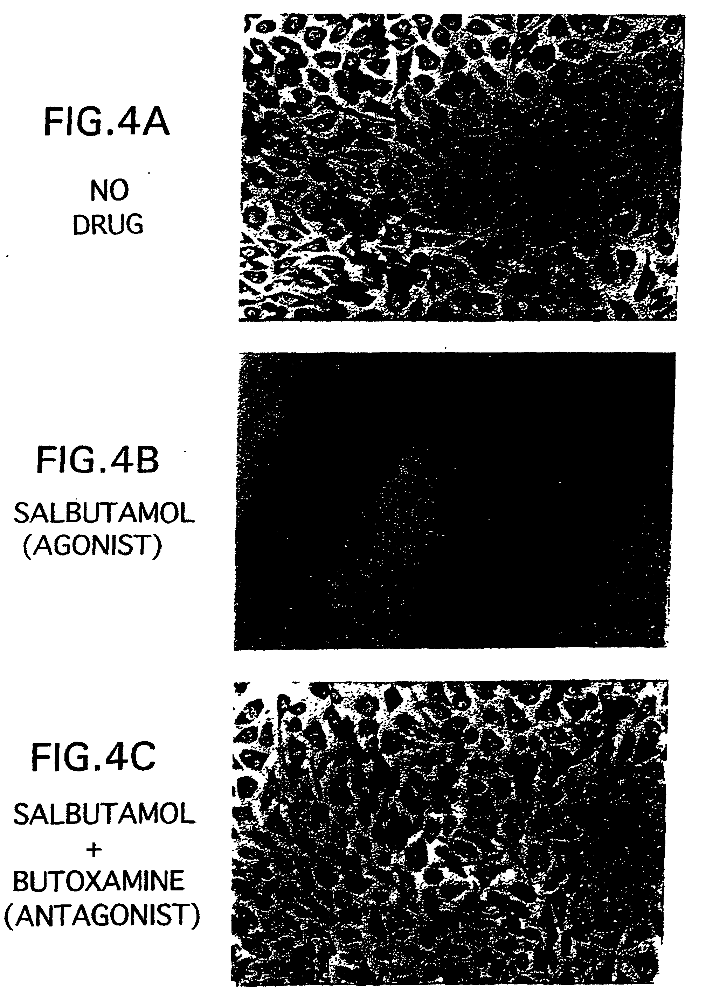

*Figure 4* is a photograph of CHO cells showing the effect of the β2-adrenergic receptor agonist salbutamol on the morphology and alignment of CHO cells expressing the human β2-adrenergic receptor.

### Detailed Description of the Invention

This invention provides methods for identifying G-protein coupled receptor (GPCR) agonists or antagonists, i.e., screening assays for agents that stimulate or inhibit the activity of a GPCR. The methods of the invention are functional bioassays. The methods are based, at least in part, on the discovery of detectable changes in cellular metabolism that occur in indicator cells expressing the GPCR when the indicator cells are contacted with a receptor agonist or antagonist. In various embodiments of the invention, these detectable changes in cellular metabolism include changes in cell viability, cell proliferation or cell morphology. For example, one embodiment of the method of the invention is based, at least in part, on the discovery that an indicator cell expressing a GPCR displays decreased cell viability and/or proliferation in the presence of a receptor agonist. Moreover, cell viability and/or proliferation in the presence of a receptor agonist can be restored by the additional presence of a receptor antagonist. Another embodiment of the method of the invention is based, at least in part, on the discovery that an indicator cell expressing a GPCR displays detectable changes in cell morphology in the presence of a receptor agonist and that these morphological changes are reversed in the presence of a receptor antagonist.

The methods of the invention are simple, rapid and high-throughput assays that allow for the efficient screening of either individual compounds or libraries of compounds. Furthermore, these methods are widely applicable to different GPCRs and can even be used to identify ligands for "orphan" receptors of unknown function. Accordingly, the methods of the invention can be used both to investigate GPCR function and to identify compounds useful for manipulating or altering GPCR activity, e.g., for therapeutic purposes. In a preferred embodiment of the invention, mammalian cells are used as indicator cells in the screening assays, thereby allowing for identification of agonist or antagonists of mammalian GPCRs in the context of a mammalian cellular environment.

Other aspects of the invention pertain to methods for preparing indicator cells useful in the screening assays of the invention and compositions, e.g., cells and vectors, used in the assays. The various aspects of the invention are described in further detail below.

In one embodiment, the invention provides a method for identifying an agonist or antagonist of a G-protein coupled receptor comprising:
a) contacting a population of indicator cells with a test composition containing at least one test compound,
b) measuring at least one parameter of cellular metabolism of the indicator cells; and
c) identifying the at least one test compound of the test composition as an agonist or an antagonist of the G-protein coupled receptor.

As used herein, the term "G-protein coupled receptor" (or "GPCR") refers to a receptor that, when expressed by a cell, associates with a G-protein (e.g., a protein composed of α, β and γ subunits and which hydrolyzes GTP). Preferably, the GPCR is a "seven transmembrane segment receptor" (or "7 TMS receptor"), which refers to a protein that structurally comprises seven hydrophobic transmembrane spanning regions.

Examples of GPCRs suitable for use in the methods of the invention include the luteinizing hormone releasing hormone (LHRH) (also known as gonadotropin releasing hormone, GnRH) receptor, the M1 muscarinic receptor and the β2-adrenergic receptor. Other preferred receptors include opioid receptors, endothelin receptors, angiotensin receptors, neuropeptide Y receptors and serotonin K receptors.

Additionally, groups of GPCRs have been classified according the G-protein to which they couple (i.e., associate with) intracellularly. Accordingly, in one embodiment, the GPCR used in the screening assay couples to (i.e., associates with) a Gq/11 G-protein. Examples of GPCRs that couple to a Gq/11 G-protein include the following receptors: LHRH (=GnRH), acetylcholine (m1, 3 and 5 subtypes), M1 muscarinic, adenosine 1, α-adrenergic (a1A, a1B and a1C subtypes), angiotensin (AT1A subtype), bombesin (BB1 and BB2 subtypes), bradykinin (B2 subtype), C5a, cholycystokinin (CCKa and CCKb subtypes), endothelin (Eta and Etb subtypes), glutamate (mGlul, 5 subtypes), 5HT (2A, B and C subtypes), histamine (H1 subtype), neurotensin, neurokinin (NK2, 3 subtypes), oxytocin, thyrotropin releasing hormone (TRH), thyroid stimulating hormone (TSH), thromoboxane A2 and vasopressin (V1a subtypes).

In another embodiment, the GPCR used in the screening assay couples to (i.e., associates with) a Gₛ G-protein. Examples of GPCRs that couple to a Gₛ G-protein include the following receptors: β2-adrenergic, cardiac β-adrenergic, histamine (H2 subtype), thyrotropin, growth hormone releasing factor, adrenocorticotropic hormone (ACTH), 5HT₄, follicle stimulating hormone (FSH), thyroid stimulating hormone (TSH), GLP-1, glucagon, domamine₅ (D₅), dopamine₁ (D₁), calcitonin, adenosine2_{β} (A2_{β}), vasopressin₂, vasoactive intestinal polypeptide and parathyroid hormone.

In another embodiment, the GPCR used in the screening assay couples to (i.e., associates with) a Gᵢ G-protein. Examples of GPCRs that couple to a Gᵢ G-protein include the following receptors: 5HT (1A, 1B, 1D and 1F subtypes), mGlutamineR (2, 3 subtypes), dopamine₄ (D4), dopamine-2 (D₂) cannabinoid, adenosine₃ (A₃), somatostatin (4, 3 subtypes), µ-opiod, δ-opiod, κ-opiod, neuropeptide Y (1, 2 subtypes).

In yet another embodiment, the GPCR is what has been referred to in the art as an "orphan" receptor. An orphan receptor is a GPCR that is structurally similar to other GPCRs but for which the natural ligand is unknown prior to use of the orphan receptor in the screening assay of the invention. In this latter embodiment, the screening assay can thus be used to identify test compounds as ligands of the orphan receptor. For example, naturally-occurring substances can be screened in the assay to investigate whether they are natural ligands for the orphan receptor. Accordingly, in addition to allowing for agonists and antagonists of known GPCRs to be identified, the bioassays of the invention also are useful for investigating the function of orphan GPCRs by identifying ligands for these receptors.

As used herein, a receptor "antagonist" refers to an agent that inhibits the activity of a GPCR, whereas a receptor "agonist" refers to an agent that stimulates the activity of a GPCR. Inhibition or stimulation of receptor activity by an antagonist or agonist, respectively, may be partial or complete.

As used herein, the term "population of indicator cells" refers to a multiplicity of cells that express at least one GPCR of interest (i.e., the GPCR for which a receptor agonist or antagonist is to be identified). An indicator cell "expresses" a GPCR when the GPCR is present on a membrane of the indicator cells. The indicator cells may naturally express the GPCR of interest (also referred to as "endogenous" expression) or, more preferably, the indicator cells express the GPCR of interest because a a nucleic acid molecule that encodes the receptor has been introduced into the indicator cells, thereby allowing for expression of the receptor on the membrane of the cells (also referred to as "exogenous" expression).

Cells which endogenously express a GPCR are well known in the art and can be used in the screening assay of the invention. However, more preferably, an indicator cell for use in the assay is prepared by introducing into the cell a nucleic acid (e.g., DNA) encoding the GPCR of interest such that the GPCR is expressed on a membrane of the cell. Preferably, the indicator cell does not express the GPCR of interest prior to introducing the GPCR-encoding nucleic acid into the cell. The GPCR-encoding nucleic acid introduced into the cell is in a form suitable for expression of the receptor on a membrane of the cell, meaning that the nucleic acid contains all of the coding and regulatory sequences required for transcription and translation of the nucleic acid encoding the GPCR, which may include promoters, enhancers and polyadenylation signals, and sequences necessary for transport of the receptor to the surface of the cell, including N-terminal leader sequences. In a preferred embodiment, the nucleic acid introduced into the cells is a recombinant expression vector carrying a cDNA encoding the GPCR. In recombinant expression vectors, the regulatory functions responsible for transcription and/or translation of the cDNA are often provided by viral sequences. Examples of commonly used viral promoters and/or enhancers include those derived from polyoma, Adenovirus 2, cytomegalovirus and Simian Virus 40, and retroviral LTRs. Suitable expression vectors for expression of a GPCR in an indicator cell are well known in the art and many are commercially available. The recombinant expression vector can be, for example, a plasmid vector or a viral vector (e.g., a retroviral vector, an adenoviral vector or an adeno-associated viral vector). Expression of the GPCR on the surface of the indicator cell can be accomplished, for example, by including the native sequence of the GPCR in the cDNA carried by the recombinant expression vector, and, preferably, by adding a heterologous signal sequence (i.e., from another protein) for the native GPCR sequence to change the intracellular processing pathway of the GPCR. It is known in the art that use of a heterologous leader sequence can improve the efficiency of expression of a protein in a host cell. For example, the leader sequence from tissue plasminogen activator (tPA) can be added to the native sequence of a GPCR.

In addition to introducing into the host cell a nucleic acid molecule encoding a GPCR, additional nucleic acid molecules encoding other gene products may also be introduced, if desired, to modulate the responsiveness of the host cells (e.g., to increase or decrease their senstivity to agonists or antagonists). For example, a nucleic acid molecule encoding a G-protein of interest (e.g., Gq/11, Gₛ, Gᵢ, etc.) can be cotransfected into the host cell such that the G-protein is expressed in the host cell (e.g., either to increase the amount of expressed G-protein in a host cell which endogenously expresses that same G-protein, or alternatively, to allow for expression of a particular G-protein in a host cell which does not endogenously express that particular G-protein).

A nucleic acid encoding a GPCR (e.g., a recombinant expression vector) can be introduced into cells by a variety of well-known cell transfection techniques useful for this purpose, including electroporation, calcium-phosphate precipitation, DEAE-dextran treatment, lipofection, microinjection and infection with viral vectors. Suitable methods for introducing nucleic acid into cells can be found in Sambrook et al. (Molecular Cloning: A Laboratory Manual, 2nd Edition, Cold Spring Harbor Laboratory press (1989)) and other laboratory manuals, and kits for transfecting cells are commercially available. Cells may be transiently transfected or, more preferably, are stably transfected with the GPCR-encoding nucleic acid. Stable transfection can be accomplished by use of an autonomously replicating expression vector (e.g., an episomal vector) or by selecting those cells which have integrated the GPCR-encoding nucleic acid into their genomes. Since typically only a small percentage of transfected cells integrate the introduced nucleic acid into their genome, it is advantageous to transfect a nucleic acid encoding a selectable marker into the indicator cells along with the GPCR-encoding nucleic acid. Preferred selectable markers include those which confer resistance to drugs such as G418, hygromycin and methotrexate. Selectable markers may be introduced on the same nucleic acid molecule (e.g., plasmid) encoding the GPCR or may be introduced on a separate nucleic acid molecule (e.g., plasmid).

In a preferred embodiment, the indicator cells used in the methods of the invention are mammalian cells. The ability to use mammalian cells in the screening assay of the invention may be particularly advantageous when the GPCR of interest is from humans or other mammals (e.g., GPCRs of therapeutic relevance) since the test compound(s) is assayed against the receptor within the context of a mammalian cell environment. This is in contrast to assay systems that utilize yeast (e.g., PCT Application No. WO 94/23025; and Jeansonne, M.E. et al. (1994) *Proc. Soc. Exp. Biol. Med*. 206:35-44) or pigment cells from amphibians or reptiles (e.g., PCT Application No. WO 92/01810; and Lerner, M.R. (1994) *Trends in Neuroscience* 17:142-146). Non-limiting examples of suitable mammalian cell lines that can be used to prepare indicator cells for use in the methods of the invention include Chinese Hamster Ovary (CHO) cells, COS cells, 3T3 cells, HeLa cells and 293 cells. The most preferred cells for use in the methods of the invention are CHO cells. Other similarly suitable cells will be apparent to those skilled in the art. Mammalian cell lines from which indicator cells can be prepared are available from the American Type Culture Collection, Rockville, MD.

As used herein, the term "test composition" is intended to encompass material that includes at least one "test compound", which refers to a compound to be assayed to determine whether it is a receptor agonist or antagonist. A test composition may contain a single test compound or multiple test compounds. For example, the test composition can include a library of test compounds, e.g., a combinatorial library of peptides. A library of test compounds can be screened in pools, and the pools that test positive for a receptor agonist or antagonist can be subdivided and rescreened. This process then can be repeated as necessary until a single positive test compound is identified from the library.

Optionally, the test composition can include additional substances. Additional substances that can be included in the test composition include known receptor agonists and known receptor antagonists. For example, a known receptor agonist can be included in the test composition to elicit a metabolic response from the indicator cells and a test compound can be identified as a receptor antagonist based upon its ability to reverse or inhibit the metabolic response elicited by the receptor agonist. Similarly, a known receptor antagonist can be included in the test composition to elicit a metabolic response from the indicator cells and a test compound can be identified as a receptor agonist based upon its ability to reverse or inhibit the metabolic response elicited by the receptor antagonist. Other substances that can be included in the test composition include agents that alter the metabolism of second messengers in the indicator cells. Examples of such agents include phorbol esters (e.g., phorbol myristate acetate), calcium ionophores (e.g., A23187), phosphodiesterase inhibitors (e.g., isobutyl-methylxanthine) and staurosporine. Such agents can be included in the test composition to alter (e.g., increase or decrease) the sensitivity of the indicator cells to the test compound(s) and/or a known receptor agonist or antagonist.

As used herein, the term "parameter of cellular metabolism" is intended to include detectable indicators of cellular responses that are regulated, at least in part, by a GPCR expressed by the indicator cell. Examples of parameters of cellular metabolism that can be measured or determined in the assays of the invention include cellular viability, cellular proliferation and cellular morphology (explained in further detail below). A test compound is identified as a receptor agonist or antagonist based upon its causing a change in at least one parameter of cellular metabolism of the indicator cells when the test compound is contacted with the indicator cells, as compared to the cellular metabolism of the indicator cells in the absence of the test compound.

In one embodiment of the invention, an agonist or antagonist of a GPCR is identified based upon its ability to alter the viability and/or proliferation of a population of indicator cells. Accordingly, in one embodiment, the invention provides a method for identifying an agonist or an antagonist of a G-protein coupled receptor, comprising:
a) contacting a population of indicator cells with a test composition containing at least one test compound;
b) measuring viability or proliferation of the indicator cells; and
c) identifying the at least one test compound as an agonist or an antagonist of the G-protein coupled receptor.

As described in further detail in Examples 2 and 3, GPCR-expressing indicator cells of the invention exhibit decreased cell viability and/or proliferation when contacted with a receptor agonist. Moreover, this decreased cell viability and/or proliferation of the indicator cells can be counteracted, or reversed, by further contact of the indicator cells with a receptor antagonist. Therefore, a test compound can be identified as an agonist of a GPCR based upon its ability to decrease the viability and/or proliferation of a population of indicator cells when contacted with the cells, as compared to the viability and/or proliferation of the indicator cells in the absence of the test compound.

In another embodiment, the test composition comprises a test compound(s) and at least one known receptor agonist or antagonist. For example, a test compound can be identified as an antagonist of a GPCR based upon its ability to increase the viability and/or proliferation of a population of indicator cells when contacted with the cells in the presence of a receptor agonist, as compared to the viability and/or proliferation of the indicator cells in the presence of the receptor agonist alone. Accordingly, in another embodiment, the invention provides a method for identifying an antagonist of a G-protein coupled receptor, comprising:
a) contacting a population of indicator cells with a test composition containing at least one test compound and at least one known receptor agonist;
b) measuring viability or proliferation of the indicator cells; and
c) identifying the at least one test compound as an antagonist of the G-protein coupled receptor.
Since, in this system, the identified antagonist has a positive effect on the indicator cells (i.e., the antagonist restores the viability and/or proliferative response of the cells), it is unlikely that false positives will be identified by the assay. This is in contrast to an assay system wherein antagonist stimulation leads to cell death, in which case compounds that are non-specifically toxic to the cells would be selected in addition to compounds that act specifically as receptor antagonists.

The viability or proliferation of the indicator cells can be measured by one of several suitable techniques well known in the art. For example, cell viability can be determined by cell counts (e.g., with a haemocytometer) of cells, preferably treated with a dye such as trypan blue (which is excluded from viable cells) or by measuring an enzymatic activity in the indicator cells that correlates with cell viability, such as reduction of a tetrazolium salt (e.g., 3,(4,4-dimethylthiazol-2-yl)2,5-diphenyl-tetrazolium bromide [MTT]), which can be detected spectrophotometrically (see Examples 2 and 3). Additionally, cell viability can be determined using such indicator dyes as propidium iodide, acridine orange and Hoechst 33342. Cell proliferation can also be determined, for example, by tritiated thymidine uptake. For mammalian cells, measurement of cell viability or proliferation of the indicator cells is typically performed at about 2 to 7 days after contact with the test composition.

A preferred GPCR for use in this assay system is LHRH-R. As demonstrated in Example 2, when an LHRH-R-expressing Chinese Hamster Ovary (CHO) cell is contacted with a known LHRH-R agonist, the reduction of MTT by the cells (as an indicator of cell viability) is decreased. Moreover, when the LHRH-R-expressing CHO cells are contacted with both the receptor agonist and a known receptor antagonist, the decreased viability of the cells induced by the agonist is reversed. A test compound(s) can similarly be screened against such cells and the viability and/or proliferation of the cells measured to identify the test compound(s) as a receptor agonist or antagonist. Known LHRH-R agonists or antagonists, which can be included in the test composition (e.g., a known agonist may be included in the test composition to identify test compounds as receptor antagonists) are commercially available (e.g., from Sigma Chemical Co., St. Louis, MO). Preferred LHRH-R agonists include LHRH analogues such as [D-His⁶]-LHRH, whereas a preferred LHRH-R antagonist is amide.

Another preferred GPCR for use in this assay system is the M1 muscarinic receptor. As demonstrated in Example 3, when an M1 muscarinic receptor-expressing CHO cell is contacted with a known M1 muscarinic receptor agonist, the reduction of MTT by the cells (as an indicator of cell viability) is decreased. Moreover, when the M1 muscarinic receptor-expressing CHO cells are contacted with both the receptor agonist and a known receptor antagonist, the decreased viability of the cells induced by the agonist is reversed. A test compound(s) can similarly be screening against such cells and the viability and/or proliferation of the cells measured to identify the test compound(s) as a receptor agonist or antagonist. Known M1 muscarinic receptor agonists or antagonists, which can be included in the test composition (e.g., a known agonist may be included in the test composition to identify test compounds as receptor antagonists) are commercially available (e.g., from Sigma Chemical Co., St. Louis, MO). A preferred M1 muscarinic receptor agonist is carbachol, whereas a preferred M1 muscarinic receptor antagonist is pirenzepine.

In a preferred embodiment, when viability or proliferation of the indicator cells is used as the metabolic parameter based upon which a test compound is identified as a receptor agonist or antagonist, the GPCR expressed by the indicator cells couples to a Gq/11 G-protein. In addition to the LHRH receptor described above, other examples of GPCRs that couple to a Gq/11 G-protein include the following receptors: M1 muscarinic, acetylcholine (m1, 3 and 5 subtypes), adenosine 1, α-adrenergic (a1A, a1B and a1C subtypes), angiotensin (AT1A subtype), bombesin (BB1 and BB2 subtypes), bradykinin (B2 subtype), C5a, cholycystokinin (CCKa and CCKb subtypes), endothelin (Eta and Etb subtypes), glutamate (mGlu1, 5 subtypes), SHT (2A, B and C subtypes), histamine (H1 subtype), neurotensin, neurokinin (NK2, 3 subtypes), oxytocin, thyrotropin releasing hormone (TRH), TSH, thromoboxane A2 and vasopressin (V1a subtypes).

In yet another embodiment, the GPCR used in this functional bioassay is a chimeric GPCR. The chimeric receptor comprises at least one ligand binding domain of a first seven transmembrane segment (7 TMS) receptor and at least one signal transduction domain of a second seven transmembrane segment (7 TMS) receptor. In a preferred embodiment, the signal transduction domain of the second seven transmembrane segment (7TMS) is selected such that the chimeric receptor couples to a Gq/11 G-protein in the indicator cells. In other embodiments, the signal transduction domain of the second seven transmembrane segment (7TMS) is selected such that the chimeric receptor couples to another type of G-protein, e.g., Gs or Gi, in the indicator cells.

Regarding the structure of 7 TMS receptors, the transmembrane segments will be referred to herein as TMS I-VII (from N-terminus to C-terminus), the three intracellular loops will be referred to as IC 1 (linking TMS I and TMS II), IC 2 (linking TMS III and TMS IV) and IC 3 (linking TMS V and VI) and the three extracellular loops will be referred to as EC I (linking TMS II and TMS III), EC 2 (linking TMS IV and V) and EC 3 (linking VI and VII). The "ligand binding domain" of a 7 TMS receptor refers to the portion(s) of the receptor that is necessary for recognition of a ligand by the receptor, whereas the "signal transduction domain" of a 7 TMS receptor refers to the portion(s) of the receptor that is necessary for signal transduction by the receptor and in particular for coupling of the receptor to a specific G-protein (e.g., Gq/11).

The construction and expression of certain chimeric 7 TMS receptors, composed of portions of one 7 TMS receptor fused to portions of a second 7 TMS receptor, have been described in the art. For example, chimeras have been constructed between α2- and β2-adrenergic receptors (see e.g., Kobilika, B.K. et al. (1988) *Science* 240:1310-1316; ), between α1- and β2-adrenergic receptors (see e.g., Cotecchia, S. et al. (1990) *Proc. Natl. Acad. Sci. USA* 87:2896-2900) and between M1- and M2-muscarinic receptors (see e.g., Kubo, T. et al. (1988) *FEBS Letters* 241:119-125). These studies have demonstrated that distinct structural determinants of a 7 TMS receptor contribute to the ligand binding properties of the receptor or to the G-protein coupling properties of the receptor. In particular, determinants involved in G-protein recognition have been mapped to intracellular loop 3 (IC 3), which connects TMS V and TMS V1. Accordingly, in one embodiment, the "at least one signal transduction domain of a second 7 TMS receptor" of the chimeric receptor comprises at least intracellular loop 3 (IC 3) of a 7 TMS receptor that couples to a G-protein of interest, e.g., a Gq/11, Gₛ or Gᵢ G-protein.

Preferably, IC 3 from an LHRH receptor (which couples to Gq/11) is used. More preferably, IC 3 from the human LHRH receptor is used. The nucleotide and amino acid sequences of mouse and human LHRH-R are disclosed in PCT Publication No. WO 94/00590 by S.C. Sealfon. According to the amino acid sequence of human LHRH-R disclosed therein, IC 3 corresponds to about amino acid residues 234 to 268. Therefore, to construct a chimeric GPCR comprising IC 3 of human LHRH-R, a nucleotide sequence encoding amino acid residues 234 to 268 of human LHRH-R is exchanged for the nucleotide sequence encoding IC 3 of another 7 TMS receptor using standard recombinant DNA techniques (such as the polymerase chain reaction, restriction endonuclease digestion, DNA ligation and the like). For example, to construct a β2-adrenergic receptor (β2-AR)/LHRH-R chimeric receptor, the region of the β2-AR cDNA encoding IC 3 is deleted and replaced with a nucleotide sequence encoding IC 3 of LHRH-R. This chimeric cDNA can be cloned into a recombinant expression vector and introduced into host cells by standard techniques, as described above, to create indicator cells expressing the chimeric receptor for use in the bioassays of the invention.

In addition to IC 3, the chimeric receptor may contain additional portions of the second 7 TMS receptor involved in signal transduction, such as IC 1 and/or IC 2. For example, when the second 7 TMS receptor of the chimera is human LHRH-R, amino acid residues 62-76 (corresponding to IC 1) and/or 140-156 (corresponding to IC 2) of human LHRH-R can be exchanged for the equivalent IC 1 and/or IC 2 domains of the first 7 TMS receptor of the chimera. Moreover, a carboxy terminal region may be deleted or added to the chimera. For example, when the first 7 TMS receptor of the chimera (which contributes the ligand binding domain(s)) normally incudes a carboxy terminal region and the second 7 TMS receptor of the chimera (which contributes the signal transduction domain(s)) is LHRH-R, the carboxy terminal region of the first 7 TMS receptor may be deleted or omitted from the chimera since the LHRH receptor normally lacks a carboxy terminal region.

In another embodiment of the invention, an agonist or antagonist of a GPCR is identified based upon its ability to alter the cellular morphology of a population of indicator cells. Accordingly, the invention provides a method for identifying an agonist or an antagonist of a G-protein coupled receptor, comprising:
a) contacting a population of indicator cells with a test composition containing at least one test compound;
b) determining the morphology of the indicator cells; and
c) identifying the at least one test compound as an agonist or an antagonist of the G-protein coupled receptor.

It has been observed that GPCR-expressing indicator cells of the invention exhibit changes in cellular morphology when contacted with a receptor agonist. For example, indicator cells such as CHO cells expressing GPCRs that couple to a Gq/11 G-protein (e.g., LHRH-R) become more rounded and detach from the culture dish when contacted with a receptor agonist. Additionally, indicator cells expressing GPCRs that couple to a Gₛ G-protein (e.g., β2-adrenergic receptor) become more spindled and elongated when contacted with a receptor agonist. Moreover, such changes in cellular morphology can be counteracted, or reversed, by further contact of the indicator cells with a receptor antagonist. Therefore, a test compound can be identified as an agonist of a GPCR based upon its ability to alter the cellular morphology of indicator cells, as compared to the cellular morphology in the absence of the test compound, when the cells are contacted with the test compound.

In another embodiment of this bioassay, the test composition includes a test compound(s) and at least one known receptor agonist or antagonist. For example, a test compound can be identified as an antagonist of a GPCR based upon its ability, in the presence of a receptor agonist, to return the indicator cells to their original morphology when cultured in the absence of the agonist. Accordingly, in another embodiment, the invention provides a method for identifying an antagonist of a G-protein coupled receptor, comprising:
a) contacting a population of indicator cells with a test composition containing at least one test compound and at least one known receptor agonist;
b) determining the cellular morphology of the indicator cells; and
c) identifying the at least one test compound as an antagonist of the G-protein coupled receptor.

As used herein, the term "cellular morphology" refers generally to the overall shape of a cell. Cellular morphology of indicator cells can be determined by standard light microscopy. Moreover, digital imaging of the cells can be performed to quantitate parameters of cellular morphology, such as cell surface area. For mammalian cells, determination of the morphology is typically performed at about 24 to 72 hours after contact with the test composition.

GPCRs as previously described herein can be used in this bioassay wherein receptor agonists or antagonists are identified based upon their effects on the morphology of the indicator cells. For example, in various embodiments, the GPCR can couple to a Gq/11 G-protein or a Gₛ protein, the GPCR can be a chimeric receptor or the GPCR can be an "orphan" receptor.

Another aspect of the invention pertains to methods for preparing cells that express recombinant GPCRs. These cells are suitable for use as indicator cells in the bioassays of the invention. The methods of the invention for preparing GPCR-expressing cells are based, at least in part, on the discovery that stimulation of a recombinant GPCR expressed on a host cell with a receptor agonist can lead to decreased cell viability and/or decreased cell proliferation. Accordingly, for improved generation of GPCR-expressing cells, stimulation of the GPCR by receptor agonists is inhibited or avoided. Thus, the invention provides an improved method for preparing a cell that expresses a recombinant G-protein coupled receptor on a membrane of the cell comprising introducing into the cell a nucleic acid molecule that encodes the receptor such that the receptor is expressed on a membrane of the cell, under culture conditions in which stimulation of the receptor by receptor agonists is inhibited.

As used herein, the term "recombinant G-protein coupled receptor" refers to a GPCR that is encoded by an exogenous nucleic acid molecule introduced into a host cell (e.g., the GPCR is encoded by a recombinant expression vector that is transfected into the host cell). Preferably, the host cell lacks endogenous expression of the GPCR. The recombinant GPCR may be derived from the same species as the host cell or from a different species than the host cell (e.g., a human recombinant GPCR can be expressed on a non-human host cell, such as a CHO, COS or 3T3 cell, etc.).

In one embodiment of the method for generating a cell expressing a recombinant GPCR, the "culture conditions in which stimulation of the receptor by receptor agonists is inhibited" comprise culture of the cell in a medium that contains a receptor antagonist. Known receptor antagonists for many different GPCRs are commercially available. For example, for LHRH-R, the known receptor antagonist antide can be included in the medium or, for the M1 muscarinic receptor, the known receptor antagonist pirenzepine can be included in the medium.

In another embodiment of the method for generating a cell expressing a recombinant GPCR, the "culture conditions in which stimulation of the receptor by receptor agonists is inhibited" comprise culture of the cell in a medium that lacks receptor agonists. Media that lack receptor agonists may be prepared, for example, using serum from which receptor agonists have been removed, e.g., by dialysis. For example, dialyzed fetal bovine serum can be used as the source of growth factors for the cells.

In the method of the invention for preparing a GPCR-expressing cell, the cell can be maintained under the culture conditions in which stimulation of the receptor by receptor agonists is inhibited until stable expression of the receptor on a membrane of the cell is achieved. Accordingly, the invention further provides methods for preparing stable populations of cells expressing recombinant GPCRs. As used herein, a "stable" cell population refers to cells whose expression of a recombinant GPCR remains essentially unchanged over time in culture (e.g., due to integration of the GPCR-encoding nucleic acid into the genome of the cell). Preferably, expression of the GPCR by the cell is stable for at least one month, more preferably six months, even more preferably one year and most preferably, indefinitely.

Another aspect of the invention, therefore, pertains to stable populations of cells expressing recombinant GPCRs. The invention provides improved cell populations that can be used, for example, as indicator cells in the bioassays of the invention. In one embodiment, these cells comprise a stable population of cells expressing a recombinant G-protein coupled receptor in a culture medium under culture conditions in which stimulation of the receptor by receptor agonists is inhibited. The "culture conditions in which stimulation of the receptor by receptor agonists is inhibited" can comprise, for example, a culture medium that contains a receptor antagonist or a culture medium that lacks receptor agonists (e.g., a culture medium in which the serum therein has been dialyzed), as described above.

Yet another aspect of the invention pertains to nucleic acid compositions encoding chimeric GPCRs and host cells expressing chimeric GPCRs. For example, the invention provides an isolated nucleic acid molecule encoding a chimeric G-protein coupled receptor (GPCR). The chimeric GPCR comprises at least one ligand binding domain of a first seven transmembrane segment receptor and at least one signal transduction domain of a second seven transmembrane segment receptor such that the chimeric GPCR couples to a Gq/11 G-protein when the chimeric GPCR is expressed in a host cell. Preferably, the second 7 TMS receptor is an LHRH receptor. More preferably, the second 7 TMS receptor is human LHRH receptor. The first 7 TMS receptor of the chimera preferably is a GPCR that normally does not couple to a Gq/11 G-protein. For example, the first 7 TMS receptor can be β2-adrenergic receptor (which couples to Gₛ). Nucleic acids encoding chimeric receptors can be prepared using standard molecular biology techniques by exchanging nucleotide sequences encoding a portion(s) of the second 7 TMS receptor for the nucleotide sequences encoding the corresponding portion(s) of the first 7 TMS receptor (as described above). For example, in a preferred embodiment, portions of a cDNA encoding the first 7 TMS receptor are replaced with portions of the LHRH-R cDNA comprising the signal transduction domain(s) of LHRH-R. Thus, in one embodiment, the nucleic acid of the invention encodes a chimeric receptor comprising intracellular loop 3 of a GPCR that couples to Gq/11, preferably IC 3 of LHRH-R. In another embodiment, the nucleic acid of the invention encodes a chimeric receptor comprising intracellular loops 1, 2 and 3 of a GPCR that couples to Gq/11 (e.g., preferably IC 1, 2 and 3 of LHRH-R). In yet another embodiment, the nucleic acid of the invention encodes a chimeric receptor comprising intracellular loops 1, 2 and 3 of a GPCR that couples to Cq/11 (e.g., preferably LHRH-R) and furthermore, the carboxy terminal domain of the chimeric receptor is deleted. Recombinant expression vectors comprising a nucleic acid molecule encoding a chimeric GPCR and host cells into which such a recombinant expression vector has been introduced are also encompassed by the invention. Such host cells expressing a chimeric GPCR can be used as indicator cells in the bioassays of the invention.

Another aspect of the invention pertains to a Chinese Hamster Ovary (CHO) cell that expresses a recombinant human luteinizing hormone releasing hormone receptor. Yet another aspect of the invention pertains to a Chinese Hamster Ovary cell that expresses a recombinant chimeric G-protein coupled receptor. The chimeric receptor comprises at least one ligand binding domain of a first seven transmembrane segment receptor and at least one signal transduction domain of a second seven transmembrane segment receptor, wherein the chimeric receptor couples to a Gq/11 G-protein in the cell. Preferably, the second 7 TMS receptor of the chimera is an LHRH receptor. More preferably, the second 7 TMS receptor of the chimera is a human LHRH receptor. The CHO cells of the invention also can be used as indicator cells in the bioassays of the invention.

The methods of the invention for identifying agonists or antagonists of GPCRs are useful for identifying compounds that may be therapeutically useful in the treatment of disease conditions that involve aberrant activity of a GPCR. Many human diseases have been traced to a mutation in a GPCR (reviewed in Coughlin, S.R. (1994) *Curr. Op. Cell. Biol*. 6:191-197). Moreover, many human diseases are currently treated with known agonists or antagonists of GPCRs. Examples include LHRH agonist such as leuprolide, gonadorelin and nafarelin, which have been used to treat prostate and breast carcinomas, uterine leimyomatas, endometriosis, precocious puberty and nontumorous ovarian hyperandrogenic syndrome, the cardiac β-adrenergic receptor antagonist propranolol, which has been used to treat hypertension, angina pectoris and psychiatric disorders, the pulmonary β2-adrenergic receptor agonist metaproterenol, which has been used as a bronchodilator and the histamine 2 receptor antagonist cimetidine, which has been used to treat ulcers and idiopathic urticaria. Other examples of GPCRs and their potential clinical indications are as follows: acetylcholine (motion sickness); adenosine 1 (renal disease, sleep apnea; cognitive disorders); α-adrenergic (hypertension, benign prostatic hypertrophy, impotence); angiotensin (renal disease); bombesin (small cell lung cancer; smooth muscle contraction); bradykinin (inflammation); C5a (inflammation); choly-cystokinin (panic attack; analgesia); endothelin (hypertension, myocardial infarction, ulcers, asthma, renal failure); and glutamate (memory & learning). Additional and improved receptor agonists and antagonists can be identified using the screening assay of the invention. Moreover, ligands of orphan GPCRs of unknown function can be identified, thereby allowing for an increased understanding of the physiological role for these orphan GPCRs and perhaps the use of agonists or antagonists therefor as therapeutic targets.

This invention is further illustrated by the following examples which should not be construed as limiting. The contents of all references, patents and published patent applications cited throughout this application are hereby incorporated by reference.

### EXAMPLE 1: Construction of an Indicator Cell Expressing a Luteinizing Hormone Releasing Hormone Receptor

To create an indicator cell that expresses a luteinizing hormone releasing hormone receptor (LHRH-R), suitable for use in the screening assays ofthe invention, a cDNA encoding human LHRH-R was cloned into a recombinant expression vector, the vector was transfected into Chinese Hamster Ovary (CHO) cells and stably transfected clones were selected as follows.

A cDNA encoding human LHRH-R protein of the amino acid sequence (the nucleotide and amino acid sequences of which are disclosed in PCT Publication No. WO 94/00590 by S.C. Sealfon), was cloned into a recombination expression vector using standard molecular biology techniques. In the vector, expression of LHRH-R was directed by the SV40 enhancer and the adenovirus major late promoter. Additionally, the vector contained a dihydofolate reductase (DHFR) gene as a selectable marker.

Dihydrofolate reductase-deficient CHO cells (DUKX B1 cells; available from the American Type Culture Collection, Rockville, MD, Catalog No. ATCC CRL 9010) were transfected with the above-described LHRH-R expression vector by standard lipofection methods (e.g., using the Lipofectin™ reagent, commercially available from Gibco/BRL, Gaithersburg, MD). To select stably transfected clones, the transfected cells were maintained in alpha MEM media/10 % dialyzed fetal calf serum that contained increasing amounts of methotrexate. Stable clones were selected based on their acquired methotrexate resistance.

### EXAMPLE 2: Assays for LHRH-R Antagonists and Agonists

An LHRH-R-expressing CHO cell clone, as described in Example 1, was used in a bioassay to identify LHRH-R antagonist or agonists as follows. The cells were plated into the wells of a 96-well plate (2500 cells/well) and cultured in the presence of a known LHRH-R agonist alone ([D-His⁶]-LHRH) (commercially available from Sigma Chemical Co., St. Louis, MO) at concentrations between 1 picomolar and 10 nanomolar, or in the presence of a known LHRH-R antagonist alone (antide) (commercially available from Sigma Chemical Co., St. Louis, MO), at concentrations between 1 picomolar and 100 nanomolar, or in the presence of both the agonist and the antagonist, at various combinations of concentrations. As a control, LHRH-R-expressing CHO cells were cultured in the absence of any additional substances. As another control, untransfected CHO DUKX B 1 cells were cultured with various concentrations of agonist ([D-His⁶]-LHRH) alone or antagonist (amide) alone.

After 5 days of culture, cell viability was assessed using the viability indicator 3,(4,4-dimethylthiazol-2-yl)2,5-diphenyl-tetrazolium bromide (MTT). (See Shearman. M.S. et al. (1994) *Proc. Natl. Acad Sci. USA* 91:1470-1474; Hansen, M.B. et al. (1989) *J. Immun. Methods* 119:203-210). MTT (commercially available from Sigma Chemical Co., St. Louis, MO) is a chromogenic substrate that in viable cells is enzymatically reduced from a yellow color to a blue color, which can be detected spectrophotometrically. To measure cell viability in the presence of the agonist and/or antagonist, MTT (at a final concentration of 1 mg/ml) was added to the cells for the final 2.75 hours of culture at 37 °C. Following incubation with MTT, the media was removed and the cells were lysed in isopropanol/0.4N HCl with agitation. The absorbance of each well at 570 nm was measured to quantitate viable cells. Alternatively, MTT was solubilized by addition of 50 % N,N-dimethyl formamide/20 % sodium dodecyl sulfate added directly to the media in the wells and viable cells were likewise quantitated by measuring absorbance at 570 nm.

The results of a representative example of a bioassay using LHRH-R-expressing CHO cells are shown graphically in Figure 1B, whereas the results with control untransfected CHO cells are shown graphically in Figure 1A. The data illustrated in Figure 1B demonstrate that the viability of the LHRH-R-expressing CHO cells was not affected by the presence of increasing amounts of receptor antagonist alone, whereas cell viability was markedly reduced in the presence of increasing amounts of receptor agonist alone (indicated by reduced absorbance at 570 nm). Notably, this reduced cell viability in the presence of the receptor agonist was reversed when the receptor antagonist was present together with the receptor agonist. This effect was dose dependent, with higher concentrations of receptor antagonist leading to greater cell viability. In contrast, neither the agonist or the antagonist had any effect on the viability of untransfected CHO cells (Figure 1A), demonstrating that this phenomenon is dependent upon the expression of LHRH-R on the CHO cells.

The results described herein demonstrate that increased viability of the LHRH-R-expressing CHO cells in the presence of a receptor agonist and a second compound, as compared to viability of the cells in the presence of the agonist alone, indicates that the second compound is a receptor antagonist. Furthermore, the results demonstrate that decreased viability of the LHRH-R-expressing CHO cells in the presence of a receptor antagonist and a second compound or in the presence of the second compound alone, as compared to viability of the cells in the presence of the antagonist alone or in the absence of any compounds, indicates that the second compound is a receptor agonist.

### EXAMPLE 3: Assays for M1 Muscarinic Receptor Antagonists and Agonists

CHO cells transfected to express an M1 muscarinic receptor (e.g., M1 WT3 cells, available from the American Type Culture Collection, Rockville, MD, Catalog No. CRL 1985) were used in a bioassay to identify antagonist or agonists of the M1 muscarinic receptor essentially as described in Example 2. Briefly, the cells were plated into the wells of a 96-well plate (2500 cells/well) and cultured in the presence of a known M1 muscarinic receptor agonist alone (carbachol) (also known as carbamylcholine chloride, commercially available from Sigma Chemical Co., St. Louis, MO), at concentrations between 100 nanomolar and 10 millimolar, or in the presence of a known M1 muscarinic receptor antagonist alone (pirenzepine) (commercially available from Sigma Chemical Co., St. Louis, MO), at concentrations between 100 nanomolar and 1 millimolar, or in the presence of both the agonist and the antagonist, at various combinations of concentrations. As a control, M1 muscarinic receptor-expressing CHO cells were cultured in the absence of any additional substances. As another control, untransfected CHO DUKX B1 cells were cultured with various concentrations of agonist (carbachol) alone or antagonist (pirenzepine) alone.

After 5 days of culture, cell viability was measured using the viability indicator MTT as described in Example 2. The results of a representative example of a bioassay using the M1 muscarinic receptor-expressing CHO cells are shown graphically in Figure 2B, whereas the results with control untransfected CHO cells are shown graphically in Figure 2A. Similar to the results observed with the LHRH-R-expressing cells described in Example 2, the data illustrated in Figure 2B demonstrate that the viability of the M1 muscarinic receptor-expressing CHO cells was not affected by the presence of increasing amounts of receptor antagonist (pirenzepine) alone, whereas cell viability was markedly reduced in the presence of increasing amounts of receptor agonist (carbachol) alone (indicated by reduced absorbance at 570 nm). Notably, this reduced cell viability in the presence of the receptor agonist was reversed when the receptor antagonist was present together with the receptor agonist. This effect was dose dependent, with higher concentrations of receptor antagonist leading to greater cell viability. In contrast, neither the agonist or the antagonist had little effect on the viability of untransfected CHO cells (Figure 2A), demonstrating that this phenomenon was dependent upon the expression of M1 muscarinic receptor on the CHO cells.

The results described herein demonstrate that increased viability of the M1 muscarinic receptor-expressing CHO cells in the presence of a receptor agonist and a second compound, as compared to viability of the cells in the presence of the agonist alone, indicates that the second compound is a receptor antagonist Furthermore, the results demonstrate that decreased viability of the M1 muscarinic receptor-expressing CHO cells in the presence of a receptor antagonist and a second compound or in the presence of the second compound alone, as compared to viability of the cells in the presence of the antagonist alone or in the absence of any compounds, indicates that the second compound is a receptor agonist.

### EXAMPLE 4: Screening of a Library of Test Compounds

An LHRH-R-expressing cell line, prepared as described in Example 1, was incubated with a library of compounds to identify agonists or antagonists of the receptor. The library was comprised of at least 100 different peptide compounds produced using combinatorial methods, divided into 10 sample pools of at least 10 compounds each. The final concentration of peptide was 1.5 µM in each well of the 96-well plate. Two of the samples were "spiked" with either the LHRH-R antagonist antide (in sample 6 at 1.5 µM) or the LHRH-R agonist D-His⁶-LHRH (in sample 4 at 1.5 µM).

Cells were assayed in the "antagonist mode" (i.e., conditions which allow for identification of receptor antagonists) by coincubating test compounds in the presence of 1 nM D-His⁶-LHRH, or in the "agonist mode" (i.e., conditions which allow the identification of receptor agonists) by incubating test compounds alone with no other additions to the cell culture. After 5 days of culture, cell viability was assessed as described in Example 2. The results, shown graphically in Figure 3, demonstrate that the assay system in the "agonist mode" readily identified the sample spiked with the known LHRH agonist (sample 4, with D-His⁶-LHRH), whereas the assay system in the "antagonist mode" readily identified the sample spiked with the known LHRH antagonist (sample 6, with antide). Furthermore, the presence of the pool of combinatorially-produced peptides did not interfere with the assay, indicating that complex mixtures of compounds can be screened using the system of the invention.

### EXAMPLE 5: Description of Cell Morphology Changes

A human β2-adrenergic receptor-expressing cell line, prepared as described in Example 1, was incubated with either 1) 1 nM salbutamol (a known β2-adrenergic receptor agonist) or 2) 10 nM butoxamine (a known β2-adrenergic receptor antagonist) together with salbutamol, in complete growth medium. Several days later, cells were analyzed by photomicroscopy to determine the effect of treatment on cell morphology. A representative photograph of the results with cells treated with salbutamol alone is shown in Figure 4. Salbutamol induced both a change in cell shape (i.e., elongation of the cells) and an alignment of the cells with each other. Consequently, the total are of the culture surface covered by the cells was greatly reduced by exposing the cells to salbutamol. Cells exposed to both salbutamol and butoxamine were indistinguishable from control cells, demonstrating antagonism of this effect by the receptor antagonist. Assessment of the agonist or antagonist properties of individual compounds or libraries of compounds can be achieved by, for example, microscopic observation of the cells, or alternatively, by video digitizing techniques to quantify cell shape changes and/or cell alignment changes. Butoxamine alone produced no detectable change in cell shape or alignment, nor did salbutamol on untransfected CHO cells that did not express the recombinant β2-adrenergic receptor.

### EQUIVALENTS

Those skilled in the art will recognize, or be able to ascertain using no more than routine experimentation, many equivalents to the specific embodiments of the invention described herein. Such equivalents are intended to be encompassed by the following claims.

## Claims

1. A method for identifying an agonist or an antagonist of a G-protein coupled receptor, comprising:
a) contacting a population of indicator cells, *e.g*, which are mammalian cells, with a test composition containing at least one test compound, wherein the indicator cells are not CHO cells;
b) measuring viability or proliferation of the indicator cells; and
c) identifying the at least one test compound as an agonist or an antagonist of the G-protein coupled receptor.

2. The method of claim 1, wherein a nucleic acid molecule that encodes the receptor has been introduced into the indicator cells such that the receptor is expressed on a membrane of the cells.

3. The method of claim 2, wherein the G-protein coupled receptor is a chimeric receptor comprising at least one ligand binding domain of a first seven transmembrane segment receptor and at least one signal transduction domain of a second seven transmembrane segment receptor such that the chimeric receptor couples to a Gq/11 G-protein in the indicator cells.

4. The method of claim 1, wherein the at least one test compound is identified as an agonist of the G-protein coupled receptor based upon the ability of the at least one test compound to decrease viability or proliferation of the indicator cells when contacted with the indicator cells, as compared to viability or proliferation of the indicator cells in the absence of the at least one test compound.

5. The method of claim 1, wherein the at least one test compound is identified as an antagonist of the G-protein coupled receptor based upon the ability of the at least one test compound to increase viability or proliferation of the indicator cells when contacted with the indicator cells in the presence of a receptor agonist, as compared to viability or proliferation of the indicator cells in the presence of the receptor agonist alone.

6. A method for identifying an agonist or an antagonist of a G-protein coupled receptor, comprising:
a) contacting a population of indicator cells, e.g. which are mammalian cells, with a test composition containing at least one test compound;
b) determining the morphology of the indicator cells; and
c) identifying the at least one test compound as an agonist or an antagonist of the G-protein coupled receptor.

7. The method of claim 6, wherein a nucleic acid molecule that encodes the receptor has been introduced into the indicator cells such that the receptor is expressed on a membrane of the cells.

8. A method for identifying an agonist or an antagonist of a G-protein coupled receptor, comprising:
a) contacting a population of indicator cells, *e.g*., which are mammalian cells, with a test composition containing at least one test compound;
b) measuring viability or proliferation of the indicator cells; and
c) identifying the at least one test compound as an agonist or an antagonist of the G-protein coupled receptor, wherein the G-protein coupled receptor is not an LHRH receptor.

9. The method of claim 8, wherein a nucleic acid molecule that encodes the receptor has been introduced into the indicator cells such that the receptor is expressed on a membrane of the cells.

10. The method of claim 9, wherein the G-protein coupled receptor is a chimeric receptor comprising at least one ligand binding domain of a first seven transmembrane segment receptor and at least one signal transduction domain of a second seven transmembrane segment receptor such that the chimeric receptor couples to a Gq/11 G-protein in the indicator cells.

11. The method of claim 8, wherein the at least one test compound is identified as an agonist of the G-protein coupled receptor based upon the ability of the at least one test compound to decrease viability or proliferation of the indicator cells when contacted with the indicator cells, as compared to viability or proliferation of the indicator cells in the absence of the at least one test compound.

12. The method of claim 8, wherein the at least one test compound is identified as an antagonist of the G-protein coupled receptor based upon the ability of the at least one test compound to increase viability or proliferation of the indicator cells when contacted with the indicator cells in the presence of a receptor agonist, as compared to viability or proliferation of the indicator cells in the presence of the receptor agonist alone.

13. The method of claim 1, claim 6, or claim 8, wherein the G-protein coupled receptor couples to a Gq/11 G-protein and, for example, the G-protein coupled receptor is a luteinizing hormone releasing hormone receptor or an M1 muscarinic receptor.

14. The method of claim 1, claim 6, or claim 8, wherein the G-protein coupled receptor couples to a Gₛ G-protein or a G, G-protein and, for example, the G-protein coupled receptor is a β2-adrenergic receptor.

15. The method of claim 1, claim 6, or claim 8, wherein the test composition comprises a library of test compounds.

16. The method of claim 1, claim 6, or claim 8, wherein the test composition further comprises at least one known receptor agonist or at least one known receptor antagonist.

17. The method of claim 1, claim 6, or claim 8, wherein the test composition further comprises at least one known receptor agonist to thereby identify a receptor antagonist.

18. The method of claim 1, claim 6, or claim 8, wherein the test composition further comprises at least one agent that alters the metabolism of at least one second messenger in the indicator cells.

19. The method of claim 1, claim 6, or claim 8, wherein the natural ligand for the G-protein coupled receptor is unknown.

## Patentansprüche

1. Ein Verfahren zur Identifizierung eines Agonisten oder eines Antagonisten eines G-Protein gekoppelten Rezeptors, umfassend:
a) Inkontaktbringen einer Population an Indikatorzellen, z.B. die Säugerzellen sind, mit einer Test-Zusammensetzung, die wenigstens eine Test-Verbindung enthält, worin die Indikatorzellen keine CHO-Zellen sind;
b) Messen der Lebensfähigkeit oder Proliferation der Indikatorzellen; und
c) Identifizieren der wenigstens einen Test-Verbindung als ein Agonist oder ein Antagonist des G-Protein gekoppelten Rezeptors.

2. Das Verfahren nach Anspruch 1, worin ein Nucleinsäure-Molekül, das den Rezeptor codiert, in die Indikatorzellen so eingeführt worden ist, dass der Rezeptor auf einer Membran der Zellen exprimiert ist.

3. Das Verfahren nach Anspruch 2, worin der G-Protein gekoppelte Rezeptor ein chimärer Rezeptor ist, umfassend wenigstens eine Ligand-bindende Domäne eines ersten Sieben-Transmembran-Segment Rezeptors und wenigstens eine Signaltransduktionsdomäne eines zweiten Sieben-Transmembran-Segment Rezeptors, so dass der chimäre Rezeptor an ein Gq/11 G-Protein in den Indikatorzellen koppelt.

4. Das Verfahren nach Anspruch 1, worin die wenigstens eine Test-Verbindung als ein Agonist des G-Protein gekoppelten Rezeptors identifiziert wird, beruhend auf der Fähigkeit der wenigstens einen Test-Verbindung, die Lebensfähigkeit oder Proliferation der Indikatorzellen zu vermindern, wenn sie mit den Indikatorzellen in Kontakt gebracht ist, im Vergleich mit der Lebensfähigkeit oder Proliferation der Indikatorzellen in Abwesenheit der wenigstens einen Test-Verbindung.

5. Das Verfahren nach Anspruch 1, worin die wenigstens eine Test-Verbindung als ein Antagonist des G-Protein gekoppelten Rezeptors identifiziert wird, beruhend auf der Fähigkeit der wenigstens einen Test-Verbindung, die Lebensfähigkeit oder Proliferation der Indikatorzellen zu erhöhen, wenn sie mit den Indikatorzellen in Anwesenheit eines Rezeptor-Agonisten in Kontakt gebracht ist, im Vergleich mit der Lebensfähigkeit oder Proliferation der Indikatorzellen in Anwesenheit nur des Rezeptor-Agonisten.

6. Ein Verfahren zur Identifizierung eines Agonisten oder eines Antagonisten eines G-Protein gekoppelten Rezeptors, umfassend:
a) Inkontaktbringen einer Population an Indikatorzellen, z.B. die Säugerzellen sind, mit einer Test-Zusammensetzung, die wenigstens eine Test-Verbindung enthält;
b) Bestimmen der Morphologie der Indikatorzellen; und
c) Identifizieren der wenigstens einen Test-Verbindung als ein Agonist oder ein Antagonist des G-Protein gekoppelten Rezeptors.

7. Das Verfahren nach Anspruch 6, worin ein Nucleinsäure-Molekül, das den Rezeptor codiert, in die Indikatorzellen so eingeführt worden ist, dass der Rezeptor auf einer Membran der Zellen exprimiert ist.

8. Ein Verfahren zur Identifizierung eines Agonisten oder eines Antagonisten eines G-Protein gekoppelten Rezeptors, umfassend:
a) Inkontaktbringen einer Population an Indikatorzellen, z.B. die Säugerzellen sind, mit einer Test-Zusammensetzung, die wenigstens eine Test-Verbindung enthält;
b) Messen der Lebensfähigkeit oder Proliferation der Indikatorzellen; und
c) Identifizieren der wenigstens einen Test-Verbindung als ein Agonist oder ein Antagonist des G-Protein gekoppelten Rezeptors, worin der G-Protein gekoppelte Rezeptor kein LHRH-Rezeptor ist.

9. Das Verfahren nach Anspruch 8, worin ein Nucleinsäure-Molekül, das den Rezeptor codiert, in die Indikatorzellen so eingeführt worden ist, dass der Rezeptor auf einer Membran der Zellen exprimiert ist.

10. Das Verfahren nach Anspruch 9, worin der G-Protein gekoppelte Rezeptor ein chimärer Rezeptor ist, umfassend wenigstens eine Ligand-bindende Domäne eines ersten Sieben-Transmembran-Segment Rezeptors und wenigstens eine Signaltransduktionsdomäne eines zweiten Sieben-Transmembran-Segment Rezeptors, so dass der chimäre Rezeptor an ein Gq/11 G-Protein in den Indikatorzellen koppelt.

11. Das Verfahren nach Anspruch 8, worin die wenigstens eine Test-Verbindung als ein Agonist des G-Protein gekoppelten Rezeptors identifiziert wird, beruhend auf der Fähigkeit der wenigstens einen Test-Verbindung, die Lebensfähigkeit oder Proliferation der Indikatorzellen zu vermindern, wenn sie mit den Indikatorzellen in Kontakt gebracht ist, im Vergleich mit der Lebensfähigkeit oder Proliferation der Indikatorzellen in Abwesenheit der wenigstens einen Test-Verbindung.

12. Das Verfahren nach Anspruch 8, worin die wenigstens eine Test-Verbindung als ein Antagonist des G-Protein gekoppelten Rezeptors identifiziert wird, beruhend auf der Fähigkeit der wenigstens einen Test-Verbindung, die Lebensfähigkeit oder Proliferation der Indikatorzellen zu erhöhen, wenn sie mit den Indikatorzellen in Anwesenheit eines Rezeptor-Agonisten in Kontakt gebracht ist, im Vergleich mit der Lebensfähigkeit oder Proliferation der Indikatorzellen in Anwesenheit nur des Rezeptor-Agonisten.

13. Das Verfahren nach Anspruch 1, Anspruch 6 oder Anspruch 8, worin der G-Protein gekoppelte Rezeptor an ein Gq/11 G-Protein koppelt und zum Beispiel der G-Protein gekoppelte Rezeptor ein luteinisierendes Hormon abgebender Hormon Rezeptor oder ein Ml Muscarin-Rezeptor ist.

14. Das Verfahren nach Anspruch 1, Anspruch 6 oder Anspruch 8, worin der G-Protein gekoppelte Rezeptor an ein Gs G-Protein oder ein Gᵢ G-Protein koppelt und zum Beispiel der G-Protein gekoppelte Rezeptor ein β2-Adrenorezeptor ist.

15. Das Verfahren nach Anspruch 1, Anspruch 6 oder Anspruch 8, worin die Test-Zusammensetzung eine Bibliothek an Test-Verbindungen umfasst.

16. Das Verfahren nach Anspruch 1, Anspruch 6 oder Anspruch 8, worin die Test-Zusammensetzung außerdem wenigstens einen bekannten Rezeptor-Agonisten oder wenigstens einen bekannten Rezeptor-Antagonisten umfasst.

17. Das Verfahren nach Anspruch 1, Anspruch 6 oder Anspruch 8, worin die Test-Zusammensetzung außerdem wenigstens einen bekannten Rezeptor-Agonisten umfasst, um auf diese Weise einen Rezeptor-Antagonisten zu identifizieren.

18. Das Verfahren nach Anspruch 1, Anspruch 6 oder Anspruch 8, worin die Test-Zusammensetzung außerdem wenigstens ein Agens umfasst, das den Metabolismus wenigstens eines zweiten Messengers in den Indikatorzellen verändert.

19. Das Verfahren nach Anspruch 1, Anspruch 6 oder Anspruch 8, worin der natürliche Ligand für den G-Protein gekoppelten Rezeptor nicht bekannt ist.

## Revendications

1. Méthode pour identifier un agoniste ou un antagoniste d'un récepteur couplé à la protéine G, comprenant le fait de :
a) mettre en contact une population de cellules indicatrices, par exemple, qui sont des cellules de mammifère, avec une composition à tester, contenant au moins un composé à tester, où les cellules indicatrices ne sont pas des cellules CHO ;
b) mesurer la viabilité ou la prolifération des cellules indicatrices ; et
c) identifier le / au moins un composé à tester comme un agoniste ou un antagoniste du récepteur couplé à la protéine G.

2. Méthode selon la revendication 1, dans laquelle une molécule d'acide nucléique qui code le récepteur a été introduite dans les cellules indicatrices de sorte que le récepteur soit exprimé sur une membrane des cellules.

3. Méthode selon la revendication 2, dans laquelle le récepteur couplé à la protéine G est un récepteur chimérique comprenant au moins un domaine de liaison à un ligand d'un premier récepteur à sept segments transmembranaires et au moins un domaine de transduction du signal d'un second récepteur à sept segments transmembranaires de sorte que le récepteur chimérique se couple à une protéine G Gq/11 dans les cellules indicatrices.

4. Méthode selon la revendication 1, dans laquelle le / au moins un composé à tester est identifié comme un agoniste du récepteur couplé à la protéine G d'après la capacité du / d'au moins un composé à tester à réduire la viabilité ou la prolifération des cellules indicatrices lorsqu'il est en contact avec les cellules indicatrices, par comparaison avec la viabilité ou à la prolifération des cellules indicatrices en l'absence du au moins un composé à tester.

5. Méthode selon la revendication 1, dans laquelle le / au moins un composé à tester est identifié comme un antagoniste du récepteur couplé à la protéine G d'après la capacité du / d'au moins un composé à tester à augmenter la viabilité ou la prolifération des cellules indicatrices lorsqu'il est en contact avec des cellules indicatrices en présence d'un agoniste du récepteur, par comparaison avec la viabilité ou à la prolifération des cellules indicatrices en présence de l'agoniste du récepteur seul.

6. Méthode pour identifier un agoniste ou un antagoniste d'un récepteur couplé à la protéine G, comprenant le fait de :
a) mettre en contact une population de cellules indicatrices, par exemple, qui sont des cellules de mammifère, avec une composition à tester contenant au moins un composé à tester ;
b) déterminer la morphologie des cellules indicatrices ; et
c) identifier le / au moins un composé à tester comme un agoniste ou un antagoniste du récepteur couplé à la protéine G.

7. Méthode selon la revendication 6, dans laquelle une molécule d'acide nucléique qui code le récepteur a été introduite dans les cellules indicatrices de sorte que le récepteur soit exprimé sur une membrane des cellules.

8. Méthode pour identifier un agoniste ou un antagoniste d'un récepteur couplé à la protéine G, comprenant le fait de :
a) mettre en contact une population de cellules indicatrices, par exemple, qui sont des cellules de mammifère, avec une composition à tester, contenant au moins un composé à tester ;
b) mesurer la viabilité ou la prolifération des cellules indicatrices ; et
c) identifier le / au moins un composé à tester comme un agoniste ou un antagoniste du récepteur couplé à la protéine G, où le récepteur couplé à la protéine G n'est pas un récepteur de LHRH.

9. Méthode selon la revendication 8, dans laquelle une molécule d'acide nucléique qui code le récepteur a été introduite dans les cellules indicatrices de sorte que le récepteur soit exprimé sur une membrane des cellules.

10. Méthode selon la revendication 9, dans laquelle le récepteur couplé à la protéine G est un récepteur chimérique comprenant au moins un domaine de liaison à un ligand d'un premier récepteur à sept segments transmembranaires et au moins un domaine de transduction du signal d'un second récepteur à sept segments transmembranaires de sorte que le récepteur chimérique se couple à une protéine G Gq/11 dans les cellules indicatrices.

11. Méthode selon la revendication 8, dans laquelle le / au moins un composé à tester est identifié comme un agoniste du récepteur couplé à la protéine G d'après la capacité du / d'au moins un composé à tester à réduire la viabilité ou la prolifération des cellules indicatrices lorsqu'il est en contact avec les cellules indicatrices, par comparaison avec la viabilité ou à la prolifération des cellules indicatrices en l'absence du / d'au moins un composé à tester.

12. Méthode selon la revendication 8, dans laquelle le / au moins un composé à tester est identifié comme un antagoniste du récepteur couplé à la protéine G d'après la capacité du / d'au moins un composé à tester à augmenter la viabilité ou la prolifération des cellules indicatrices lorsqu'il est en contact avec des cellules indicatrices en présence d'un agoniste du récepteur, par comparaison avec la viabilité ou à la prolifération des cellules indicatrices en présence de l'agoniste du récepteur seul.

13. Méthode selon la revendication 1, la revendication 6 ou la revendication 8, dans laquelle le récepteur couplé à la protéine G se couple à une protéine G Gq/11 et, par exemple, le récepteur couplé à la protéine G est un récepteur de l"hormone libérant l'hormone lutéinisante (LHRH) ou un récepteur muscarinique M1.

14. Méthode selon la revendication 1, la revendication 6 ou la revendication 8, dans laquelle le récepteur couplé à la protéine G se couple à une protéine G Gₛ ou à une protéine G G₁ et, par exemple, le récepteur couplé à la protéine G est un récepteur adrénergique β2.

15. Méthode selon la revendication 1, la revendication 6 ou la revendication 8, dans laquelle la composition à tester comprend une banque de composés à tester.

16. Méthode selon la revendication 1, la revendication 6 ou la revendication 8, dans laquelle la composition à tester comprend en outre au moins un agoniste de récepteur connu ou au moins un antagoniste de récepteur connu.

17. Méthode selon la revendication 1, la revendication 6 ou la revendication 8, dans laquelle la composition à tester comprend en outre un agoniste de récepteur connu pour identifier ainsi un antagoniste de récepteur.

18. Méthode selon la revendication 1, la revendication 6 ou la revendication 8, dans laquelle la composition à tester comprend en outre au moins un agent qui modifie le métabolisme d'au moins un second messager dans les cellules indicatrices.

19. Méthode selon la revendication 1, la revendication 6 ou la revendication 8, dans laquelle le ligand naturel pour le récepteur couplé à la protéine G est inconnu.
